**Europäisches Patentamt**

**European Patent Office**

**Office européen des brevets**

(11) Veröffentlichungsnummer: **0 246 451**
**B1**

(12) # EUROPÄISCHE PATENTSCHRIFT

(45) Veröffentlichungstag der Patentschrift:
03.10.90

(51) Int. Cl.⁵: **A61B 5/00, G01N 33/49**

(21) Anmeldenummer: **87105665.1**

(22) Anmeldetag: **16.04.87**

(54) **Vorrichtung zur Bestimmung von Gaspartialdrücken im Blut.**

(30) Priorität: **13.05.86 DE 3616062**

(43) Veröffentlichungstag der Anmeldung:
**25.11.87 Patentblatt 87/48**

(45) Bekanntmachung des Hinweises auf die Patenterteilung:
**03.10.90 Patentblatt 90/40**

(84) Benannte Vertragsstaaten:
**DE FR GB IT**

(56) Entgegenhaltungen:
**DE-A- 2 725 757**
**GB-A- 2 100 859**
**US-A- 3 848 580**
**US-A- 4 127 111**
**US-A- 4 535 786**

(73) Patentinhaber: **Fresenius AG, Gluckensteinweg 5,
D-6380 Bad Homburg v.d.H.(DE)**

(72) Erfinder: **Polaschegg, Hans-Dietrich, Dr.,
Grünwiesenweg 9, D-6370 Oberursel 4(DE)**

(74) Vertreter: **Dr. Fuchs, Dr. Luderschmidt Dipl.-Phys. Seids,
Dr. Mehler Patentanwälte, Abraham-Lincoln-Strasse 7,
D-6200 Wiesbaden(DE)**

**Beschreibung**

Die Erfindung betrifft eine Vorrichtung zur Bestimmung von Gaspartialdrücken in Blut mit einem Filter, der durch eine semipermeable Membran in zwei Kammern geteilt ist, mit einer extrakorporalen Blutzuführung, an die eine Kammer angeschlossen ist, und mit wenigstens einem Sensor zur Bestimmung des Gaspartialdrucks, der über eine Leitung mit der anderen Kammer des Filters verbunden ist.

Die kontinuierliche Verfolgung der Säure-Basen-Parameter im Blut ist insbesondere bei Operationen unter Benutzung einer Herz-Lungenmaschine, aber auch während einer extrakorporalen Membranoxygenation (ECMO) von wesentlicher Bedeutung. Dabei werden die partiellen Gasdrücke von Sauerstoff und $CO_2$ sowie der pH-Wert gemessen.

Weiterhin ist eine Messung dieser Parameter stromauf und stromab des Oxygenators wesentlich für die Beurteilung der Effektivität bzw. eines eventuellen Leistungsabfalls des Oxygenators. So kann hierdurch zwischen einer durch einen Leistungsabfall des Oxygenators verursachten Änderung der Blutgaspartialdrücke und einer metabolisch verursachten Änderung unterschieden werden.

Zur Gewinnung dieser Daten werden üblicherweise dem extrakorporalen Kreislauf in bestimmten Zeitabständen Blutproben entnommen, die anschließend in einem Blutgasanalysator analysiert werden. Dies ist jedoch mit einem erheblichen personellen und materiellen Aufwand verbunden. Insofern wurden Sensoren entwickelt, die direkt in den extrakorporalen Blutkreislauf eingeschaltet sind, somit unmittelbar mit dem Blut in Berührung kommen, so daß hierdurch die Blutgase im extrakorporalen Kreislauf kontinuierlich registriert werden können.

Bei der Anwendung dieser kontinuierlich arbeitenden, direkt mit Blut in Berührung stehenden Sensoren besteht ein Kalibrierungsproblem, da sich sowohl der Nullpunkt als auch die Steilheit derartiger Sensoren relativ schnell verändern können. Insbesondere bei mehrere Stunden dauernden Herzoperationen oder bei der ECMO über mehrere Tage stellt dies ein ernsthaftes Problem dar, da hier keine zuverlässigen Werte mehr gewonnen werden können.

Des weiteren ist die direkte Einschaltung derartiger Sensoren in den extrakorporalen Blutkreislauf insofern problematisch, als Blut mit der Oberfläche des Sensors wechselwirken kann und die Gefahr besteht, daß die Meßfläche des Sensors mit Blutbestandteilen zugesetzt wird bzw. sich Ablagerungen auf der Meßfläche bilden, was zu Verfälschungen des Meßergebnisses führt.

Insofern wurde versucht, die Sensoren entsprechend dem üblichen Kalibrierverfahren vor der Behandlung zu kalibrieren und während der Behandlung in bestimmten Zeitabständen Blutproben zu entnehmen, die anschließend mit einem konventionellen Blutgasanalysator überprüft werden. Abgesehen von möglichen methodischen Fehlern und der Umständlichkeit des Verfahrens erlaubt diese Methode nicht die Prüfung oder den Abgleich der Sensorsteilheit, weil hierzu zwei Messungen bei unterschiedlichen Partialdrücken erforderlich wären.

Daneben besteht bei derartigen Behandlungen unter Zuhilfenahme eines extrakorporalen Kreislaufes auch die zusätzliche Notwendigkeit, die Konzentrationen der Blutelektrolyte zu messen, was weitere Probenentnahmen erforderlich macht.

So ist beispielsweise aus Biomedical Business International, Dec. 12, 1985 Vol. VIII, No 23/24, S. 232 ein Gerät bekannt, das sowohl Blutgase als auch Elektrolyte zugleich kontinuierlich messen kann. Dazu wird mit einem kontinuierlich arbeitenden Probenahmergerät eine geringe Menge Blut dem extrakorporalen Kreislauf oder dem Patienten direkt entnommen und im Analysegerät kontinuierlich analysiert. Dieser Vorgang kann automatisch in bestimmten Zeitabständen unterbrochen werden, worauf über eine Ventilanordnung dem Gerät Kalibrierlösung zur erneuten Einstellung zugeführt wird.

Nachteilig an dieser Anordnung ist jedoch, daß die Probeentnahme direkt im extrakoporalen Kreislauf oder dem Patienten vorgenommen wird, was aus Sterilitätsgründen nicht unproblematisch ist. Des weiteren wird dem Kreislauf Vollblut abgenommen, das anschließend mit einer gerinnungshemmenden Substanz in ausreichender Menge versetzt werden muß, so daß durch die Zugabe dieser Substanz zur Vollblutprobe das Gasgleichgewicht in der Probe gestört wird, was zu Verfälschungen des Meßergebnisses führen kann.

Insgesamt besteht bei diesem bekannten Gerät ein erhöhtes Kontaminationsrisiko durch die direkte Verbindung des Probenahmegerätes mit dem extrakorporalen Kreislauf.

Des weiteren ist aus der Standardliteratur (vergl. Oswald Müller-Plathe: Säure-Basen-Haushalt und Blutgase, 2. Auflage, Klinische Chemie in Einzeldarstellungen Band 1. H. Breuer, H. Büttner, D. Stamm, Hrsg; Georg Thieme-Verlag Stuttgart, 1982, Seite 148 ff) zu entnehmen, daß nach der herrschenden Lehrmeinung die Messung von Blutgasen im Plasma oder Serum zu gravierenden Meßfehlern führt. So wird im Plasma schon kurz nach der Probennahme und Abtrennung der roten Blutkörperchen regelmäßig ein Sauerstoffpartialdruck gemessen, der dem von Luftsauerstoff gleicht. Zur Abtrennung der Erythrozyten und zur Gewinnung des Plasmas wird das Vollblut mittels der Zentrifugation einige Minuten behandelt, wobei anschließend das überstehende Plasma mittels einer Spritze abgezogen und sofort in einem Blutgasanalysator vermessen wird. Es zeigt sich jedoch, daß bereits nach dieser kurzen Zentrifugationszeit der Sauerstoffgehalt um den Faktor 2 - 3 angestiegen ist. Des weiteren ist der $CO_2$-Partialdruck um ca. 20 % gefallen, was mit einer Hebung des pH-Wertes einhergeht.

Infolgedessen wurde bisher die Bestimmung der Blutgase im Plasma als nicht durchführbar angesehen.

Aus der DE-OS 27 25 757 ist eine Vorrichtung der eingangs erwähnten Art bekannt, bei der Blut durch einen Dialysefilter geführt wird, das auf der anderen Seite von einer Dialysierflüssigkeit durchflossen wird. Die zu untersuchenden Stoffe diffundieren durch die Poren der Membran des Dialysefilters von der Blutseite auf die Dialysierflüssigkeitsseite und werden dort mit der Dialysierflüssigkeit ab zum Analysator gefördert. Um überhaupt eine quantitative Messung durchführen zu können, müssen sämtliche Verfahrensparameter konstant sein, was jedoch nicht der Fall ist. So belegt sich die Membran mit einer Sekundärschicht aus Proteinen auf der Blutseite, was den Diffusionswiderstand bis zum Faktor 5 erhöht. Desgleichen schwankt der Elektrolytgehalt des Bluts von Proband zu Proband und auch innerhalb der Meßzeit, so daß Konzentrationsgefälle zwischen der Blut- und Dialysierflüssigkeitsseite vorliegen, die ebenfalls den Meßvorgang stark stören.

Des weiteren müssen sowohl das Blut als auch die Dialysierflüssigkeit mit möglichst konstanter Geschwindigkeit durch das Dialysefilter durchgeführt werden, um Verzerrungen in den Konzentrationen der zu messenden Stoffe zu verhindern. Da peristaltische Pumpen eingesetzt werden, ist dies aufgrund der auftretenden Pulsationen ebenfalls nicht gewährleistet.

Da die Diffusion der zu messenden Stoffe im wesentlichen den Meßvorgang bestimmt, müssen auch bei der Kalibrierung ensprechende Kalibrierlösungen auf der Blutseite dem extrakorporalen Kreislauf zugeführt werden, was praktisch zu nicht bewältigbaren Problemen hinsichtlich Reinigung und Sicherheit führt. Darüber hinaus werden durch die Zuführung von diesen Kalibrierlösungen die auf der Membranoberfläche abgelagerten Proteinschichten wieder abgetragen, was zwangsläufig zu einer Verfälschung der Meßergebnisse führt.

Der Erfindung liegt daher die Aufgabe zugrunde, die Vorrichtung gemäß Anspruch 1 so fortzubilden, daß im Blut enthaltene Gase quantitativ bestimmt werden können.

Des weiteren liegt der Erfindung die Aufgabe zugrunde, neben der quantitativen Bestimmung der Blutgase auch eine einfache und problemlose Kalibrierung durchzuführen, die nicht den extrakorporalen Blutkreislauf stört.

Verfahrensgemäß erfolgt die Lösung der Aufgabe dadurch, daß man Plasma, das die zu bestimmenden Gase aufweist, durch die Membran aus Blut abtrennt und unter Aufrechterhaltung des Ausschlusses von Luft in dem so erhaltenen Plasma den Partialdruck wenigstens eines Blutgases bestimmt.

Vorrichtungsgemäß erfolgt die Lösung der Aufgabe dadurch, daß die zweite Kammer des Filters einen einzigen Auslass aufweist, der mit der als erste Plasmaleitung ausgebildeten Leitung verbunden ist und daß in die erste Plasmaleitung eine Einheit zur Förderung von Plasma aus Blut durch die Membran des Membranfilters eingeschaltet ist.

Überraschenderweise wurde nunmehr festgestellt, daß Plasma, das mit Hilfe eines Membranfilters gewonnen und unmittelbar danach einem Blutgasanalysator zugeführt wurde, reproduzierbare und mit den entsprechenden Blutwerten übereinstimmende Ergebnisse brachte. Demzufolge ist es also nicht mehr nötig, unmittelbar mit Probenahmegeräten in den Blutkreislauf einzugreifen, so daß mit der erfindungsgemäßen Vorrichtung das bekannte Kontaminationsrisiko nicht mehr auftritt. Membranfilter, die in extrakorporalen Kreisläufen eingesetzt werden können, besitzen üblicherweise eine derart geringe Porengröße, daß sie als steril angesehen werden können, das heißt, das durch den Filter geführte Blut wird nicht mit Keimen kontaminiert. Wesentlich an dem erfindungsgemäßen Verfahren ist lediglich, daß das am Membranfilter abgezogene Plasma unmittelbar einem Gaspartialdrücke messenden Sensor zugeführt wird, was mit der erfindungsgemäßen Vorrichtung durchgeführt werden kann.

Da nicht mehr die Diffusion, sondern vielmehr die Gewinnung des Ultrafiltrats in Form des Plasmafiltrats die Messung bestimmt, ist auch keine Kalibrierung mehr auf der extrakorporalen Blutseite notwendig. Vielmehr kommt es lediglich darauf an, daß das an der Membran gewonnene Plasma vollständig die zweite Kammer des Dialysators und die sich daran anschließende Plasmaleitung ausfüllt, so daß eine Vermischung mit Luft unterbleibt, was ansonsten zu einer Verfälschung der Meßergebnisse führen würde.

Mit dieser erfindungsgemäßen Vorrichtung ist es in einer weiteren Ausführungsform auch möglich, neben der Bestimmung von Blutgasen auch Elektrolyte und gegebenenfalls weitere Substanzen, wie Heparin, kontinuierlich zu analysieren, ohne daß ein erhöhtes Kontaminationsrisiko eingegangen werden muß, da ein entsprechend dem Einsatzzweck gewähltes Plasmafilter zugleich als Sterilfilter wirkt.

Zur Bestimmung von Blutgasen ist der Einsatz eines Hämofilters geeignet, mit dem Filtrat von Blut abgetrennt wird. Derartige Hämofilter weisen üblicherweise eine Trenngrenze von etwa 60.000 atomare Masseneinheiten auf, halten also nicht nur die Blutkörperchen, sondern auch Eiweiße mit einem höheren Molekulargewicht, beispielsweise Albumin und gerinnungsaktive Substanzen auf der Blutseite zurück. Da diese Eiweißsubstanzen teilweise elektrisch geladen sind, kommt es zur Ausbildung einer Konzentrationsdifferenz zwischen Blut und Filtrat für die geladenen Teilchen (Ionen), selbst wenn diese aufgrund ihrer Größe die Membran frei passieren könnten (sog. Gibbs-Donnan-Effekt). Infolgedessen läßt sich die Konzentration von Ionen beim Einsatz eines derartigen Hämofilters nicht genau bestimmen.

Da Blutgase jedoch keine elektrische Ladung aufweisen, unterliegen sie nicht diesem Effekt und können somit unter Einsatz eines Hämofilters bestimmt werden.

Gemäß einer zweiten Ausführungsform kann als Membranfilter ein Plasmafilter eingesetzt werden, mit dem Plasmaeiweiße abgetrennt werden und der üblicherweise eine Trenngrenze von mehrerern Millionen atomare Masseneinheiten aufweist. Da an dem Filter keine geladenen Blutbestandteile mehr zurückgehalten werden, tritt auch keine Ionenkonzentrationsdifferenz zwischen Blut und Filtrat (Plasma) auf, so daß ein derart hergestelltes Plasma sowohl auf den Blutgasgehalt hin als auch auf den Gehalt seiner Ionen überprüft werden kann. Allerdings muß ein derart gewonnenes Plasma, das sämtliche gerinnungsaktiven Eiweiße enthält und insofern gerinnungsfähig ist, mit einer gerinnungshemmenden Substanz, beispielsweise Heparin, versehen werden. Gegebenenfalls kann also der Plasmafiltratauslaß mit einer Einrichtung zur Verabreichung einer derartigen gerinnungshemmenden Substanz verbunden werden, was üblicherweise jedoch nicht notwendig ist, da dem extrakorporalen Blutkreislauf üblicherweise Heparin zugesetzt wird und dieses den Plasmafilter wie die übrigen Plasmaeiweiße passiert. Hierdurch wird die Messung der gewünschten Parameter nicht beeinflußt. Desgleichen wird verhindert, daß sich die Sensoren bei der Messung zusetzen.

In einer dritten Ausführungsform ist es möglich, auch einen Filter einzusetzen, der die gerinnungsaktiven Substanzen weitgehend zurückhält, die übrigen Bestandteile mit geringerem Molekulargewicht jedoch durchläßt. Hierzu kann vorteilhafterweise ein Filter mit einer Ausschlußgrenze von etwa 100.000 atomare Masseeinheiten eingesetzt werden. Mit einem solchen, die gerinnungsfähigen Substanzen zurückhaltenden Filter kann die Ausbildung einer Konzentrationsdifferenz für Ionen und die Zugabe von Heparin im wesentlichen vermieden werden.

Wie bereits vorstehend erwähnt, wird das Verfahren üblicherweise an Vollblut durchgeführt, das im extrakorporalen Kreislauf dem Patienten entnommen worden ist. Dabei ist es nicht von Bedeutung, in welcher Weise das Blut dem Patienten entnommen und dem Patienten wiederum zurückgeführt wird. Hierzu können sowohl die Ein-Nadel-Methode als auch die Zwei-Nadel-Methode eingesetzt werden.

Um quantitative Ergebnisse zu erhalten, ist es notwendig, daß der Raum zwischen der Membran des Filters und dem Analysator, d. h. die zweite Kammer und die Plasmaleitung gegenüber der Atmosphäre abgeschlossen ist, um eine Vermischung des gewonnenen Plasmas mit Luft zu verhindern. Aufgrund des geringen Querschnitts der Leitung spielt dabei eine Rückdiffusion der Luft in das offene Leitungsende keine Rolle, zumal die Geschwindigkeit, mit der das Plasma gefördert wird, gegenüber dem Austausch der Gase an der Flüssigkeitsoberfläche groß ist.

Desgleichen ist es nicht wesentlich, für welchen Einsatzzweck der extrakorporale Kreislauf eröffnet worden ist. Insofern sind sämtliche Verwendungszwecke des extrakorporalen Kreislaufes denkbar, beispielsweise die Eröffnung des extrakorporalen Kreislaufes bei einer Herz-Lungen-Maschine, der extrakorporalen Membranoxygenation, der Hämodialyse, der Plasmapherese oder Plasmafiltration.

Des weiteren kann der für die Durchführung des Verfahrens eingesetzte Membranfilter stromauf und/oder stromab dieser Vorrichtungen eingesetzt werden, um die zu bestimmenden Parameter stromauf und/oder stromab dieser Anordnungen zu bestimmen. Insofern kann also wenigstens ein derartiges Membranfilter zur Bestimmung der Blutgaswerte eingesetzt werden.

Die Bestimmung der Blutgaswerte kann mit einem auf den speziellen Blutgaswert ansprechenden Sensor durchgeführt werden. Dementsprechend wird also zur Bestimmung des Sauerstoffgehalts ein den Partialdruck des Sauerstoffs bestimmender Sensor eingesetzt. In der gleichen Weise wird zur Bestimmung von $CO_2$ ein den Partialdruck von $CO_2$ bestimmender Sensor eingesetzt. Üblicherweise werden hierzu Sensoren eingesetzt, die in einer Durchflußanordnung vorgesehen sind, so daß die zu messende Probe entsprechend einem vorbestimmten Programm durch den Durchflußkanal gefördert und dort mit Hilfe der Sensoren gemessen wird.

Derartige Sensoren sind zur Bestimmung der Blutgasparameter von Blut bekannt und können dementsprechend auch zur Bestimmung der Blutgasparameter von Plasma eingesetzt werden.

Vorteilhafterweise weist ein derartiges Analysengerät zusätzlich noch einen pH-sensitiven Sensor auf, mit dem der pH-Wert des Plasmas gemessen werden kann, was für die Bestimmung des Säure-Basen-Gleichgewichts in Verbindung mit den beiden übrigen Parametern, insbesondere dem $CO_2$-Gehalt des Bluts besonders vorteilhaft ist.

In einer weiteren Ausführungsform ist zusätzlich zum vorstehenden beschrieben Blutgasanalysator ein Meßgerät zur Bestimmung von im Blut enthaltenen Elektrolyten vorgesehen. Vorteilhafterweise wird hierzu eine Durchflußanordnung eingesetzt, wie sie beispielsweise in der DE-OS 34 16 956 oder der DE-P 35 24 824 beschrieben ist, auf deren Offenbarung ausdrücklich Bezug genommen wird und die zum Gegenstand dieser Beschreibung gemacht werden.

Weitere Einzelheiten, Merkmale und Vorteile Erfindung werden anhand von zwei Ausführungsbeispielen, die in der Zeichnung dargestellt sind, beschrieben.

Es zeigen:

Fig. 1 eine prinzipielle Darstellung einer ersten Ausführungsform der erfindungsgemäßen Vorrichtung zur Bestimmung von Blutgasparametern und

Fig. 2 eine ebenfalls prinzipielle Darstellung einer zweiten Ausführungsform der erfindungsgemäßen Vorrichtung zur Bestimmung der Blutgasparameter und der Ionenkonzentration im Blut.

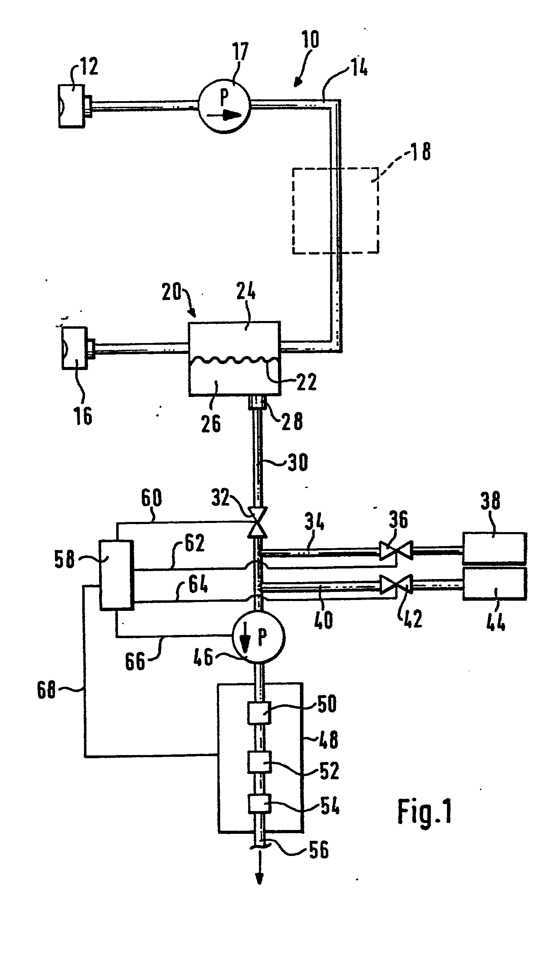

In Fig. 1 ist eine Blutgasmeßeinrichtung 10 gezeigt, der aus einem ersten Blutanschluß 12, ein sich von dem Blutanschluß 12 erstreckenden extrakorporalen Kreislauf 14 und einem zweiten Blutanschluß 16 besteht. Derartige extrakorporalen Kreisläufe sind an sich bekannt und werden üblicherweise dadurch hergestellt, daß als Blutanschlüsse 12, 16 Nadeln eingesetzt werden, die mit einem den Kreislauf 14 aufbauenden Schlauchsystem verbunden sind. In einen derartigen extrakorporalen Kreislauf 14 kann ein Blutbehandlungsgerät 18 eingeschaltet sein, das in der Fig. 1 strichliert dargestellt ist. Ein solches Blutbehandlungsgerät 18 kann beispielsweise eine Herz-Lungen-Maschine, ein Membranoxygenator, eine Hämodialysemaschine, ein Plasmapheresesystem oder ein beliebiger anderer Blutbehandlungsapparat sein.

In den extrakorporalen Kreislauf 14 ist weiterhin ein Membranfilter 20 eingeschaltet, der eine Membran 22 aufweist, die den Membranfilter in eine erste von Blut durchflossene Kammer 24 und in eine zweite von Plasma durchflossene Kammer 26 teilt. Dabei weist die Membran 22 die vorstehend erwähnten Trenneigenschaften auf, das heißt die Trenngrenze des Membranfilters 20 wird entsprechend dem Einsatzzweck gewählt. Insofern ist also die erste Kammer 24 mit der Kreislaufleitung 14 verbunden, während die zweite Kammer 26 einen Auslaß 28 aufweist, von dem eine erste Plasmaleitung 30 abgeht.

Gemäß der in Fig. 1 gezeigten Ausführungsform ist in diese Plasmaleitung ein erstes Ventil 32 eingeschaltet, dessen Funktion nachstehend erläutert wird.

Stromab des Ventils 32 ist die Plasmaleitung 30 mit einer ersten Kalibrierlösungsleitung 34 verbunden, in die ein zweites Ventil 36 eingeschaltet ist und die mit einem ersten Kalibrierlösungsbehälter 38 verbunden ist.

Weiter stromab mündet in die Plasmaleitung 30 eine zweite Kalibrierlösungsleitung 40, in die ein drittes Ventil 42 eingeschaltet ist und die mit einem zweiten Kalibrierlösungsbehälter 44 verbunden ist.

Stromab der Kalibrierlösungsleitungen 34 und 40 ist in die Plasmaleitung eine Pumpe 46 eingeschaltet, mit der das Plasma bzw. die Kalibrierlösungen durch die Plasmaleitung 30 gepumpt werden.

Das Ende der Plasmaleitung 30 mündet in einen Blutgasanalysator 48, der gemäß der in Fig. 1 gezeigten Ausführungsform als Durchflußanordnung gezeigt ist.

Dieser Blutgasanalysator 48 weist einen $PO_2$-sensitiven Sensor 50, einen $PCO_2$-sensitiven Sensor 52 und vorteilhafterweise einen pH-sensitiven Sensor 54 auf.

Nach dem Durchfließen dieses Blutgasanalysators 48 werden die gemessenen Lösungen in den Abfluß 56 geleitet.

Des weiteren weist die erfindungsgemäße Vorrichtung zur Bestimmung von Blutgasparametern eine Steuereinheit 58 auf, die über eine erste Steuerleitung 60 mit dem Ventil 32, über eine zweite Steuerleitung 62 mit dem Ventil 36, über eine dritte Steuerleitung 64 mit dem Ventil 42 und über eine vierte Steuerleitung 66 mit der Pumpe 46 verbunden ist.

Diese Steuereinheit 58 steuert entsprechend einem vorbestimmten Programm die Ventile und die Pumpe, wobei in der gewünschten Weise Plasma bzw. Kalibrierlösungen dem Blutgasanalysator 48 zugeführt werden.

Die in Fig. 1 gezeigte Vorrichtung wird auf folgende Weise betrieben:

Im extrakorporalen Kreislauf 14 zirkuliert Blut und wird durch die erste Kammer 24 des Membranfilters 20 kontinuierlich mit einer Pumpe 17 gepumpt. Um Plasma dem Membranfilter 20 zu entnehmen, wird von der Pumpe 46 nach Öffnung des Ventils 32 der zweiten Kammer 26 Plasma entnommen und dem Blutgasanalysator 48 zugeführt, indem kontinuierlich die Blutgaswerte, also der Partialdruck von Sauerstoff und Kohlendioxid sowie der pH-Wert gemessen werden.

Dabei sind während der Messung die Kalibrierlösungsleitungen 34 und 40 mit Hilfe der Ventile 36 und 42 verschlossen.

Entsprechend einem vorbestimmten Programm werden die Sensoren 50 - 54 in bestimmten Zeitintervallen kalibriert, um Fehler bei der Bestimmung der Blutgaswerte zu verhindern. Hierzu wird das Ventil 32 geschlossen und es werden nacheinander die Ventile 36 und 42 geöffnet, um eine erste und eine zweite Kalibrierlösung durch den Blutgasanalysator mit Hilfe der Pumpe 46 zu führen.

Die erste und die zweite Kalibrierlösung weisen unterschiedliche Konzentrationen von Sauerstoff und Kohlendioxyd sowie gegebenenfalls einen unterschiedlichen pH-Wert auf, um die Kalibrierung bei verschiedenen Meßpunkten durchzuführen, was Aufschluß über die Steilheit der Sensoren gibt. Dabei gibt das Steuergerät 58 über eine fünfte Steuerleitung 68, die mit dem Blutgasanalysator 48 verbunden ist, ein entsprechendes Signal ab, woraufhin der Blutgasanalysator 48 von der Meßphase in die Kalibrierphase übergeht. Diese Kalibrierphase läuft wiederum entsprechend einem bestimmten Programm im Blutgasanalysator 48 ab und ist an sich bekannt.

Nach der Kalibrierung schaltet das Steuergerät 58 erneut in die Meßphase um, wobei das Ventil 32 geöffnet und die Kalibrierlösungsventile 36 und 42 geschlossen werden. Hierauf beginnt die erneute Förderung von Plasma durch die Plasmaleitung 30 zum Blutgasanalysator 48.

Wesentlich an der Durchführung des Verfahrens ist dabei, daß das Plasma nach der Abnahme von der Membran möglichst schnell dem Blutgasanalysator zugeführt wird. Hierdurch soll vermieden werden, daß sich die Gaskonzentration im Plasma ändert, was zu einer Verfälschung der Blutgaswerte führt.

Vorteilhafterweise werden dabei nur geringe Plasmamengen an einem entsprechend dimensionierten Membranfilter 20 entnommen. So werden Schläuche mit einem entsprechend geringen Schlauchquerschnitt als Plasmaleitung 30 eingesetzt und es wird als Pumpe 46 eine peristaltische Pumpe eingesetzt, die nicht unmittelbar mit dem Plasma in Berührung gebracht wird.

Bei der Durchführung des Verfahrens können Blutgaswerte am Blutgasanalysator 48 erhalten werden, die mit den Werten übereinstimmen, die mit der üblichen Meßtechnik unter direkter Entnahme von Vollblut, das anschließend sofort im Blutgasanalysator gemessen wird, erhalten wurden. Insofern ist es mit dem erfindungsgemäßen Verfahren und der Vorrichtung zur Durchführung des Verfahrens möglich, nunmehr kontinuierlich aus einem steril abgeschlossenen extrakorporalen Blutkreislauf die gewünschten Blutgasparameter automatisch zu bestimmen, was bisher zu großen Sterilitätsproblemen geführt hat.

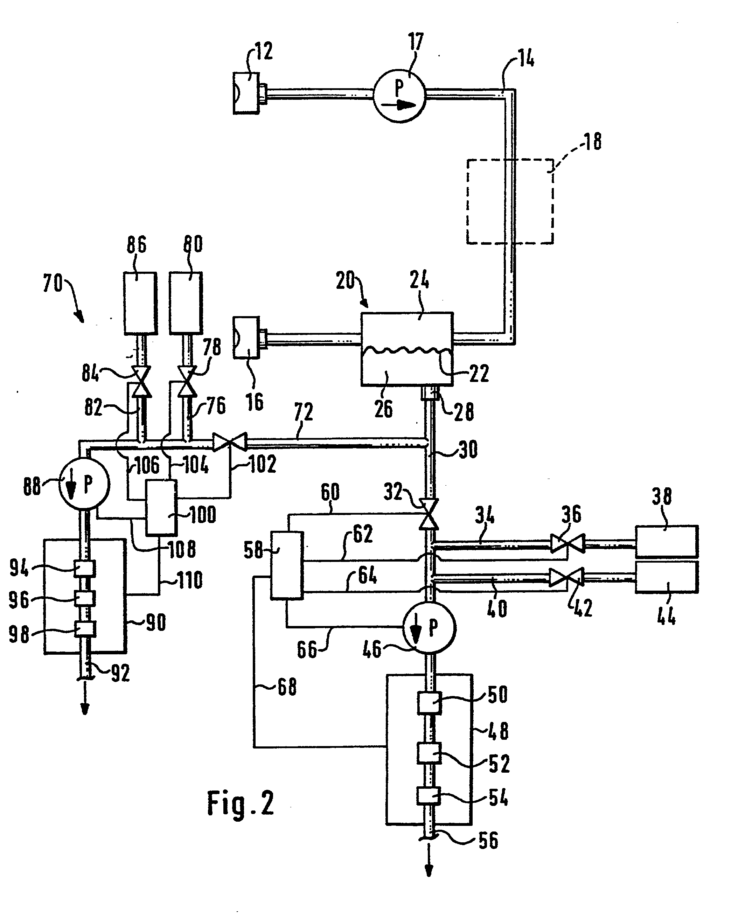

In Fig. 2 ist eine zweite Ausführungsform der erfindungsgemäßeb Vorrichtung zur Bestimmung der Blutgaswerte gezeigt, die mit 70 bezeichnet ist und sich teilweise von der ersten Ausführungsform gemäß Fig. 1 ableitet. Insofern sind gleiche Teile mit gleichen Bezugszeichen bezeichnet.

Wie aus Fig. 2 ersichtlich ist, zweigt zwischen dem Membranfilter 20 und dem ersten Ventil 32 von der Plasmaleitung 30 eine Zweigleitung 72 ab, in die ein viertes Ventil 74 eingeschaltet. Stromab des Ventils 74 geht eine dritte Kalibrierleitung 76 ab, in die ein fünftes Ventil 78 eingeschaltet ist. Das Ende der dritten Kalibrierleitung 76 ist dabei mit einem dritten Kalibrierlösungsbehälter 80 verbunden.

Weiterhin ist die Zweigleitung 72 mit einer vierten Kalibrierlösungsleitung 82 verbunden, in die ein sechstes Ventil 84 eingeschaltet ist und die an ihrem Ende einen vierten Kalibrierlösungsbehälter 86 aufweist.

Stromauf dieser Verzweigungsleitungen 76 und 82 ist in die Zweigleitung 72 eine zweite Pumpe 88 eingeschaltet, die in ihrer Funktion der Pumpe 46 entspricht. Schließlich ist das Ende der Zweigleitung 72 mit einer Vorrichtung zur Bestimmung von Ionenkonzentrationen bzw. der Leitfähigkeit 90 verbunden, die gemäß dem Ausführungsbeispiel als Durchflußanordnung gezeigt ist und die auf ihrer Austrittsseite einen Abfluß 92 aufweist. Beispielhaft ist in dieser Vorrichtung 90 ein Na-Sensor 94, ein K-Sensor 96 und eine Leitfähigkeitsmeßzelle 98 gezeigt.

Eine derartige Vorrichtung 90 ist beispielsweise in der DE-OS 34 16 956 beschrieben, auf deren Offenbarung Bezug genommen wird.

Des weiteren ist ein zweites Steuergerät 100 zur Steuerung der Ventile 74, 78 und 84, der Pumpe 88 und der Ionenmeßvorrichtung 90 vorgesehen, die entsprechende Steuerleitungen 102, 104, 106, 108 und 110 aufweist.

Das zweite Steuergerät 100 wird in ähnlicher Weise wie das erste Steuergerät 58 betrieben, so daß auf die vorstehenden Ausführungen Bezug genommen wird. Insofern wird also zur Messung der Ionenkonzentrationen bzw. der Leitfähigkeit des Plasmas das Ventil 74 geöffnet und die Pumpe 88 in Betrieb genommen. Anschließend wird das Plasma durch die Plasmaleitung 30 und die Zweigleitung 72 zur Ionenmeßeinrichtung 90 gefördert, wobei dort die entsprechenden Ionenparameter gemessen werden. Dabei ist die Messung der Parameter nicht auf die vorstehend beschriebene Na- und K-Parameter beschränkt. Insofern können auch weitere Parameter, beispielsweise Calcium, Leitfähigkeit und dergl. gemessen werden.

Entsprechend einem vorgegebenen Programm wird die Ionenmeßeinrichtung 90 in vorbestimmten Zeitintervallen kalibriert. Hierzu wird das Ventil 74 geschlossen und es werden die Ventile 78 und 84 geöffnet, wobei mit Hilfe der Pumpe 88 nacheinander zwei Kalibrierlösungen mit unterschiedlicher Zusammensetzung durch die Ionenmeßeinrichtung 90 gefördert werden. Diese Ionenkalibrierlösungen weisen entsprechend den eingesetzten Sensoren bestimmte Ionenkonzentrationen auf, die für die Eichung der Sensoren bzw. des Ionenmeßgeräts 90 eingesetzt werden müssen. Nach der Eichung und Nullpunktsfestlegung im Ionenmeßgerät 90 schaltet das Steuergerät 100 wieder in die Meßphase um, schließt dabei die Ventile 78 und 84 und öffnet anschliessend wiederum das Ventil 74. Hierauf kann erneut zu messende Plasmaflüssigkeit durch die Ionenmeßvorrichtung 90 gefördert werden.

Wie bereits vorstehend erwähnt, muß für die Messung von Ionen das Plasmafilter 20 entsprechend gewählt werden, das heißt, das Plasmafilter hat solche Proteine durchzulassen, die Ionen binden, somit also bei einem Rückhalten derartiger Proteine das Meßergebnis verfälschen würden.

Insofern ist ein Membranfilter einzusetzen, das wenigstens eine Ausschlußgrenze von etwa 100.000 atomare Masseeinheiten aufweist.

Die Ionenmeßeinrichtung 90 kann - wie vorstehend erwähnt - als vorteilhafte Ausführungsform der Blutgasmeßeinrichtung 10 eingesetzt werden. Sie kann jedoch aber auch allein ohne die Blutgasmeßeinrichtung 10 eingesetzt werden, sofern dies vorteilhaft erscheint. Sie kann aber auch kombiniert in einem einzigen Meßgerät und in einer einzigen Durchflußanordnung vorgesehen sein, das heißt, die beiden Meßgeräte 48 und 90 sind vereinigt, wobei sämtliche übrigen Teile, die eine identische Funktion aufweisen, beispielsweise die Pumpen 46 und 88 sowie die Kalibrieranordnungen zusammenfallen können. Insofern können auch Kalibrierlösungen eingesetzt werden, in denen beispielsweise der Natrium- und Kaliumgehalt, der pH-Wert sowie die Partialdrücke von Sauerstoff und Kohlendioxyd in zwei unterschiedlichen Konzentrationen bzw. Partialdrücken vorgegeben sind.

## Patentansprüche

1. Vorrichtung zur Bestimmung von Gaspartialdrücken in Blut mit einem Filter, der durch eine semipermeable Membran in zwei Kammern geteilt ist, mit einer extrakorporalen Blutzuführung, die an die eine Kammer angeschlossen ist, und mit wenigstens einem Sensor zur Bestimmung des Gaspartialdrucks, der über eine Leitung mit der anderen Kammer des Filters verbunden ist, **dadurch gekennzeichnet,** daß die zweite Kammer (26) des Filters (20) einen einzigen Auslass (28) aufweist, der mit der als erste Plasmaleitung (30) ausgebildeten Leitung verbunden ist und daß in die erste Plasmaleitung (30) eine Einheit zur Förderung von Plasma aus Blut durch die Membran (22) des Membranfilters (20) eingeschaltet ist.

2. Vorrichtung nach Anspruch 1, **dadurch gekennzeichnet,** daß die Einheit zur Förderung von Plasma eine erste Pumpe (46) ist.

3. Vorrichtung nach Anspruch 1, **dadurch gekennzeichnet,** daß stromauf der Pumpe (46) ein erstes Ventil (32) in die Plasmaleitung (30) eingeschaltet ist und daß zwischen der Pumpe (46) und dem Ventil (32) mindestens eine Kalibrierlösungsleitung (34, 40) in die Plasmaleitung (30) mündet, daß in die Kalibrierlösungsleitung (34, 40) ein Ventil (36, 42) eingeschaltet ist und daß das Ende der Kalibrierlösungsleitung (34, 40) mit einem eine Kalibrierlösung enthaltenden Behälter (38, 44) verbunden ist.

4. Vorrichtung nach Anspruch 1, **dadurch gekennzeichnet,** daß als Sensoren ein PO$_2$-Sensor (50) und ein PCO$_2$-Sensor (52) in einem Blutgasanalysator (48) vorgesehen sind.

5. Vorrichtung nach Anspruch 4, **dadurch gekennzeichnet,** daß der Blutgasanalysator (48) einen pH-Sensor (54) aufweist.

6. Vorrichtung nach einem der Ansprüche 1 bis 5, **dadurch gekennzeichnet**, daß ein Steuergerät (58) vorgesehen ist, das über Steuerleitungen (60 - 68) alternierend das in der Plasmaleitung (30) vorgesehene Ventil (32) öffnet bzw. schließt und das in der Kalibrierlösungsleitung vorgesehene Ventil (36, 42) schließt bzw. öffnet, die. Pumpe (46) aktiviert und den Blutkreisanalysator (48) alternierend in die Meß- bzw. Kalibrierphase schaltet.

7. Vorrichtung nach einem der Ansprüche 1 bis 6, **dadurch gekennzeichnet**, daß der Membranfilter (20) ein Hämofilter mit einer Ausschlußgrenze von 40.000 - 60.000 atomare Masseneinheiten oder ein Plasmafilter mit einer solchen Ausschlußgrenze ist, daß elektrisch geladene Proteine durchgelassen, gerinnungsfähige Substanzen jedoch zurückgehalten werden.

8. Vorrichtung nach einem der Ansprüche 1 bis 7, **dadurch gekennzeichnet**, daß von der Plasmaleitung (30) eine Zweigleitung (72) abzweigt, die mit einer Ionenmeßvorrichtung (90) verbunden ist und daß in die Zweigleitung (72) eine zweite Pumpe (88) eingeschaltet ist.

9. Vorrichtung nach Anspruch 8, **dadurch gekennzeichnet**, daß stromauf der Pumpe (88) in die Zweigleitung (72) ein viertes Ventil (74) eingeschaltet ist, daß zwischen dem vierten Ventil (74) und der zweiten Pumpe (88) wenigstens eine zweite Kalibrierlösungsleitung (76, 82) abzweigt, in die ein Ventil (78, 84) eingeschaltet ist und die an ihrem Ende einen Kalibrierlösungsbehälter (80, 86) aufweist und daß ein zweites Steuergerät (100) vorgesehen ist, das über Steuerleitungen (102, 110) das vierte Ventil (74), das Kalibrierlösungsventil (78, 84), die zweite Pumpe (88) und die Ionenmeßvorrichtung (90) entsprechend einem vorbestimmten Programm aktiviert.

## Claims

1. An apparatus for determining the partial pressures of gases in blood comprising a filter which is divided by a semipermeable membrane into two chambers, an extracorporeal blood conducting system connected with one of the chambers and at least one sensor for determination of the partial pressure of the gas which is connected via a duct with the other chamber of the filter, characterized in that the second chamber (26) of the filter (20) comprises one single outlet (28) which is connected with the duct designed to serve as the first plasma duct (30) and that the first plasma duct (30) comprises a unit for conveying plasma from blood through the membrane (22) of the membrane filter (20).

2. An apparatus according to claim 2, characterized in that the unit for the conveying of plasma is a first pump (46).

3. An apparatus according to claim 1, characterized in that upstream from the pump (46) a first valve (32) is mounted in the plasma duct (30) and that between the pump (46) and the valve (32) at least one calibration solution duct (34, 40) opens into the plasma duct (30), that the calibration solution duct (34, 40) comprises a valve (36, 42) and that the end of the calibration solution duct (34, 40) is connected with a container (38, 44) containing a calibration solution.

4. An apparatus according to claim 1, characterized in that as sensors a PO$_2$ sensor (50) and a PCO$_2$ sensor (52) in a blood gas analyzer (48) are provided for.

5. An apparatus according to claim 4, characterized in that the blood gas analyzer (48) comprises a pH sensor (54).

6. An apparatus according to one of claims 1 to 5, characterized in that a control unit (58) is provided for which through control lines (60–68) alternately opens or closes, respectively, the valve (32) provided for in the plasma duct (30), and which close or opens, respectively, the valve (36, 42) provided for in the calibration solution duct, activates the pump (46) and alternately switches the blood gas analyzer (48) to the measuring phase or calibration phase, respectively.

7. An apparatus according to one of claims 1 to 6, characterized in that the membrane filter (20) is a hemofilter with an exclusion limit of 40 000–60 000 atomic units of mass or a plasma filter with such an exclusion limit that electrolytically charged proteins are let through but clottable substances are retained.

8. An apparatus according to one of claims 1 to 7, characterized in that from the plasma duct (30) a branch duct (72) branches off which is connected with an ion measuring device (90) and that a second pump (88) is arranged in the branch duct (72).

9. An apparatus according to claim 8, characterized in that upstream the pump (88) a fourth valve (74) is arranged in the branch duct (72), that between the fourth valve (74) and the second pump (88) at least a second calibration solution duct (76, 82) branches off in which a valve (78, 84) is arranged and which at its end comprises a calibration solution container (80, 86), and that a second control unit (100) is provided for which through control lines (102, 110) activates the fourth valve (74), the calibration solution valve (78, 84), the second pump (88) and the ion measuring device (90) according to a given program.

## Revendications

1. Dispositif destiné à déterminer des pressions partielles de gaz dans le sang, comportant un filtre qui est partagé en deux chambres, par une membrane semi-perméable, comportant une arrivée de sang extracorporelle qui est raccordée à une chambre et comportant au moins un détecteur, en vue de la détermination de la pression partielle de gaz, qui est relié par une conduite, à l'autre chambre du filtre, caractérisé en ce que la deuxième chambre (26) du filtre (20) comporte une seule sortie (28) qui est reliée à la conduite servant de première conduite de plasma (30) et en ce que dans la première conduite de plasma (30), on monte une unité destinée à envoyer le plasma sanguin, à travers la membrane (22) du filtre à membrane (20).

2. Dispositif selon la revendication 1, caractérisé en ce que l'unité de refoulement du plasma est une première pompe (46).

3. Dispositif selon la revendication 1, caractérisé en ce qu'on monte une première vanne (32), dans la conduite de plasma (30), en amont de la pompe (46) et en ce qu'au moins une conduite de solution de calibrage (34, 40) débouche dans la conduite de plasma (30), entre la pompe (46) et la vanne (32), en ce qu'on monte une vanne (36, 42) dans la conduite de solution de calibrage (34, 40) et en ce que l'extrémité de la conduite de solution de calibrage (34, 40) est reliée à un réservoir (38, 44) contenant une solution de calibrage.

4. Dispositif selon la revendication 1, caractérisé en ce qu'on prévoit comme capteurs, un capteur PO$_2$ (50) et un capteur PCO$_2$ (52) dans un analyseur de gaz de sang (48).

5. Dispositif selon la revendication 4, caractérisé en ce que l'analyseur de gaz de sang (48) comporte un détecteur de pH (54).

6. Dispositif selon l'une quelconque des revendications 1 à 5, caractérisé en ce qu'on prévoit un appareil de commande (58) qui ouvre ou ferme alternativement la vanne (32), prévue dans la conduite de plasma (30), par des lignes de commande (60-68) et ferme ou ouvre la vanne (36, 42) prévue dans la conduite de solution de calibrage, active la pompe (46) et fait alternativement passer l'analyseur du circuit sanguin (48) en phase de mesure ou de calibrage.

7. Dispositif selon l'une quelconque des revendications 1 à 6, caractérisé en ce que le filtre à membrane (20) est un hémofiltre avec une limite de séparation de 40 000 à 60 000 unités de masse atomique ou un plasmafiltre avec une limite de séparation telle qu'il laisse passer les protéines chargées d'électrolytes, mais retient les substances coagulables.

8. Dispositif selon l'une quelconque des revendications 1 à 7, caractérisé en ce que de la conduite de plasma (30) part une conduite de dérivation (72) qui est reliée à un dispositif de mesure d'ions (90) et en ce qu'une deuxième pompe (88) est montée dans la conduite de dérivation (72).

9. Dispositif selon la revendication 8, caractérisé en ce qu'on monte une quatrième vanne (74) dans la conduite de dérivation (72), en amont de la pompe (88), en ce qu'entre la quatrième vanne (74) et la deuxième pompe (88), part une deuxième conduite de solution de calibrage (76, 82) dans laquelle est montée une vanne (78, 84) et qui, à son extrémité, comporte un réservoir de solution de calibrage (80, 86) et en ce qu'on prévoit un deuxième appareil de commande (100) qui, par des lignes de commande (102, 110), active la quatrième vanne (74), la vanne de solution de calibrage (78, 84), la deuxième pompe (88) et le dispositif de mesure d'ions (90), selon un programme prédéterminé.

Fig.1

Fig.2